# EUROPÄISCHE PATENTSCHRIFT

(11) **EP 0 332 021 B1**
(45) Veröffentlichungstag und Bekanntmachung des Hinweises auf die Patenterteilung: **01.06.1994**
(21) Anmeldenummer: 89103478.7
(22) Anmeldetag: 28.02.1989
(51) Int. Cl.: G01N 33/53, G01N 33/543

(54) **Mittel für immunchemische Tests enthaltend Aminoxide**
Agent containing amino oxide for use in immuno assays
Milieux pour immuno-essais contenant un aminoxyde

(30) Priorität: 08.03.1988 DE 3807478
(43) Veröffentlichungstag der Anmeldung: 13.09.1989
(73) Patentinhaber: BEHRINGWERKE Aktiengesellschaft, 35001 Marburg (DE)
(72) Erfinder: Schuy, Wilhelm, Dr., D-5431 Obererbach (DE)

(56) Entgegenhaltungen:
- EP-A- 0 133 272
- EP-A- 0 215 457
- EP-A- 0 234 941

## Beschreibung

Die Erfindung betrifft ein Mittel zum immunchemischen Nachweis und zur Bestimmung eines Analyten in einem biologischen Material, wobei dieses Mittel ein Aminoxid enthält.

Bekannte immunchemische Testsysteme gebrauchen Zusätze von Proteinen, Polysacchariden und/oder Tensiden, die nicht an der immunchemischen Reaktion beteiligt sind, die jedoch geeignet sind, das Ergebnis einer solchen Reaktion günstig zu beeinflussen.

In DE 36 38 767 ist ein Inkubationsmedium für festphasenimmunchemische Tests beschrieben, das Lactoferrin, foetales Kälberserum, Polyoxiethylen-20-sorbitanmonolaureat (^{R}Tween 20) und Puffersalze enthält.

In EP-A 215 457 sind ebenfalls Zusätze von Tensiden aus der Gruppe der Poloxamere, beispielsweise ^{R}Pluronic F 68 und aus der Gruppe der Poloxamine, beispielsweise ^{R}Tetronic 707 und 1107 beschrieben, wobei die drei genannten Verbindungen als vorteilhaft gegenüber ^{R}Tween 20 herausgestellt sind.

Es wurde überraschend gefunden, daß Aminoxide noch besser geeignet sind, eine immunchemische Reaktion günstig zu beeinflussen, was eine höhere Empfindlichkeit des Nachweises und der Bestimmung eines in einem auch als Probe zu bezeichnenden biologischen Material enthaltenen Analyten bewirkt.

Weiterhin wurde gefunden, daß Aminoxide als Bestandteil eines solchen Mittels bei der Vorbereitung der Probe für den Test besonders geeignet sind.

Gegenstand der Erfindung ist daher ein Mittel zum Nachweis oder zur Bestimmung eines Analyten in einem biologischen Material, enthaltend ein Aminoxid der Formel I 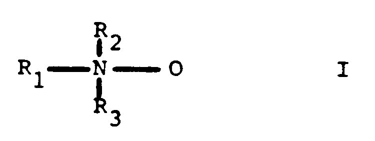
wobei
a) R₁ eine 1-Alkanoyl- oder 1-Alkenoyl-amino-propyl-Gruppe ist, wobei die Alkanoyl- oder Alkenoyl-Reste 8-18 C-Atome enthalten,oder deren Mischungen und R₂ und R₃ Methyl-, Ethyl- oder Propyl-Gruppen oder
   deren Mischungen sind, oder
b) R₁ eine Alkanyl- oder Alkenyl-Gruppe mit 8 bis 18 C-Atomen oder deren Mischungen und
   R₂ und R₃ Methyl-, Ethyl- oder Propyl-Gruppen oder deren Mischungen sind, oder
c) R₁ eine Alkanyl- oder Alkenyl-Gruppe mit 8 bis 18 C-Atomen oder deren Mischungen,
   R₂ eine Methyl-, Ethyl- oder Propylgruppe ist, und
   R₃ eine Hydroxiethyl- oder Hydroxipropylgruppe ist, oder deren Mischungen, oder
d) R₁ eine Alkanyl- oder Alkenyl-Gruppe mit 8 bis 18 C-Atomen oder deren Mischungen und
   R₂ und R₃ Hydroxiethyl- oder Hydroxipropyl-Gruppen sind, oder
e) R₁ eine Alkanyl- oder Alkenyl-Gruppe mit 8 bis 18 C-Atomen oder deren Mischungen und Oxiethyl- oder Oxipropyl-Gruppe oder deren Polykondensaten,
   R₂ und R₃ Methyl-, Ethyl-, Propyl- oder Hydroxiethyl- oder Hydroxipropyl-Gruppen oder Mischungen davon sind, oder
f) R₁ eine Alkanoyl- oder Alkenoyl-Gruppe mit 8-18 C-Atomen oder deren Mischungen und Oxiethyl- oder Oxipropyl-Gruppe oder deren Polykondensaten,
   R₂ und R₃ Methyl-, Ethyl-, Propyl- oder Hydroxiethyl-oder Hydroxipropyl- Gruppen oder Mischungen davon sind, wobei R₁ , R₂ und R₃ Amidgruppen oder Phenolreste enthalten können,
und immunchemische Reaktanten, von denen mindestens einer mit dem Analyten reagieren kann, und wobei sich diese Reaktanten ebenfalls auf oder in diesem Mittel oder zum Teil oder alle auf oder in einem weiteren Mittel befinden können.

Es sind eine Vielzahl von Mitteln enthaltend immunchemische Reaktanten und/oder bioaffine Bindungspartner bekannt.

Die Mittel werden im allgemeinen danach bezeichnet, für welche immunchemischen Verfahren sie verwandt werden.

Mittel im Sinne der Erfindung sind solche, mit denen Präzipitate als Dispersion oder in einem Gel oder Agglutinate von Partikeln erzeugt oder deren Ausbleiben bewirkt werden, oder solche, bei denen durch die immunchemische Reaktion ein Farbsignal oder eine Strahlung erzeugt oder verhindert wird.

Aus der Gruppe der letztgenannten Mittel werden solche bevorzugt, mit denen festphasenimmunchemische Tests durchgeführt werden, die bei Erzeugung einer Färbung oder einer Strahlung aus einem Enzymsubstrat als ELISA (enzyme linked immunosorbent assay) oder als Scintillations-Assay, bei Erzeugung einer Strahlung durch ein radioaktiv markiertes Isotop als Festphasen-Radioimmunoassay oder bei Erzeugung einer Fluoreszenz durch ein Fluorogen als Festphasen-Fluoreszenzassay bezeichnet werden.

Diagnostisches Mittel enthalten Antigen, Antikörper oder zugleich beide als Reaktanten, sowie andere für die Reaktanten oder auch den Analyten bioaffine Bindungspartner, beispielsweise Lektine, Komplement, Protein A oder G, sowie derivatisiertes Biotin und Avidin, wobei mindestens einer der Reaktanten mit einer Markierung versehen sein kann und gegebenenfalls Reagenzien zum Nachweis der Markierung. Außerdem kann einer der Reaktanten als Festphase vorliegen.

Eine Festphase im Sinne der Erfindung ist ein wasserunlöslicher Träger, an dem ein oder mehrere Reaktanten gebunden sind.

Träger sind beispielsweise Latexpartikel, körniges, quellbares oder nicht quellbares Material, Kugeln, Innenflächen von Röhrchen, Mikrotestplatten als besondere Ausführungsform einer Anordnung von Röhrchen und auch als saugfähige Matrix zu bezeichnende poröse Materialien.

Ein Bestandteil des erfindungsgemäßen Mittels kann eine Vorrichtung zur Aufnahme einer Probe, beispielsweise ein Gefäß zur Probenaufnahme oder die Aufnahmezone, beispielsweise eine saugfähigen Matrix, für die Probe auf einem sogenannten "trockenchemischen" Testsystem sein.
Der Bestandteil kann auch eine wässrige Lösung sein, in der auch Puffersalze und gegebenenfalls stabilisierende Zusätze wie Proteine oder Polysaccharide als ebenfalls für den Analyten stabilisierende Substanzen enthalten sind, wobei das Aminoxid in einer Konzentration von 2-80 g/l enthalten ist, bevorzugt von 5-50 g/l, besonders bevorzugt von 10-40 g/l.

Das Aminoxid kann auch in einer Vorrichtung als Bestandteil des diagnostischen Mittels enthalten sein, in der die immunchemische Reaktion stattfindet.

Die auch als Probe bezeichneten biologischen Materialien, die den Analyten enthalten, sind beispielsweise Gewebe von Biopsien oder Autopsien, Blutzellen, Serum oder Plasma, Sekrete, Liquor, Blut aus entzündeten und nicht entzündeten Gewebe, die Zufallsprodukte von Gewebe und Stoffwechselausscheidungen.

Gegenstand der Erfindung ist weiterhin ein Verfahren zur immunchemischen Bestimmung eines in einem biologischen Material enthaltenen Analyten dadurch gekennzeichnet, daß man den Analyten mit einem Aminoxid gegebenenfalls in einer wässrigen Lösung zusammenbringt und mit dem derart erhaltenen Gemisch eine immunchemische Bestimmung durchführt.

Gegenstand der Erfindung ist weiterhin die Verwendung eines Aminoxids in immunchemischen Tests.

Die biologischen Materialien werden mit dem Aminoxid zusammengebracht und können derart über längere Zeit gelagert werden, ohne daß sich der darin enthaltene Analyt in seinen immunchemischen Eigenschaften verändert.

Die biologischen Materialien können auch mit einer wässrigen Lösung, die das Aminoxid enthält, suspendiert, gelöst und/oder verdünnt und in diesem Zustand über längere Zeit gelagert werden. In dem suspendierten, gelösten und oder verdünnten Zustand werden sie ohne weitere Behandlung für den immunchemischen Nachweis oder die immunchemische Bestimmung des Analyten eingesetzt. Aminoxidhaltige wässrige Lösungen haben beim Vermischen mit viskosem Probenmaterial wie Sputum die vorteilhafte Eigenschaft, daß sie dieses Probenmaterial schneller verflüssigen und somit besser pipettierfähig machen als wässrige Lösungen von Acetylcystein und Pankreatin, Lösungen, die zur Schleimverflüssigung schon benutzt worden waren.

Bevorzugt werden immunchemische Verfahren, bei denen einer der Reaktanten in fester Phase vorliegt, wobei das wie zuvor beschriebene, behandelte Material mit der festen Phase zusammengebracht wird, gegebenenfalls zusammen mit weiteren immunchemischen Reaktanten außer denen der festen Phase und Reagenzien zum Nachweis des Analyten, wobei anschließend die feste Phase von der flüssigen Phase getrennt und der Analyt entweder an der festen oder in der flüssigen Phase bestimmt wird.

### Beispiel 1

Es wurden folgende Verbindungen in Konzentrationen von 10 g/l (a) und 40 g/l (b) (in der Tabelle sind sie als Zusätze bezeichnet) in phosphat-gepufferter Kochsalzlösung (PBS), pH 7.2, enthaltend 7.25 mmol/l Na₂HPO₄, 2.72 mmol/l KH₂PO₄, 140 mmol/l NaCl und 3 mmol/l NaN₃, gelöst:
Aminoxid WS 35, ein Produkt der Firma Goldschmidt, welches ein 1-Alkoylamino-3-dimethylamino-propan-3-N-oxid ist, wobei sich der Alkoylrest aus Mischung von Fettsäuren mit Kettenlängen von C₈ bis C₁₈ herleitet;
Cocosdimethylaminoxid der Formel 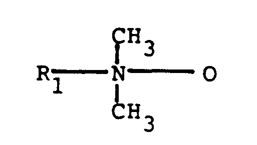
wobei R₁ ein aus dem Cocosfett erhalten Alkylrest ist, also mit Kettenlängen von C₈ bis C₁₈;
Tetradecyldimethylaminoxid;
Cocos-Bis-(2-hydroxyethyl)-aminoxid, wobei Cocos- Alkylreste sind, die aus Cocosfett erhalten wurden und Kettenlängen von C₈ bis c₁₈ haben;
^{R}Pluronic F 68, Handelsname für eine Verbindung aus der Gruppe der Poloxamere oder
^{R}Tetronic 707 und Tetronic 1107, Randelsnamen für Verbindungen aus der Gruppe der Poloxamine (Anlagerungsprodukte von Ethylenoxid und Propylenoxid an Ethylendiamin).

Aliquote von 6 µl eines Pools von Herpes simplex Virus (HSV) haltiger Bläschenflüssigkeit wurden mit jeweils 1.2 ml der zuvor beschriebenen Lösungen vermischt. Die derart erhaltenen Proben wurden bei 20-25°C bis zu 21 Tagen gelagert.

An den Tagen 0, 7, 14 und 21 wurden 150 µl entnommen und einer Bestimmung von HSV zugeführt.

Die Bestimmung wurde nach dem festphasen-2-seiten-immunchemischen Verfahren (Sandwich-ELISA) durchgeführt. Für diesen ELISA wurden folgende Materialien hergestellt:

### 1.1. Mikrotestplatten, beschichtet mit Antikörpern gegen HSV 1 und 2

Mikrotestplatten, d.h. Immunoplatten II 96 F mit rundem Boden (Firma Nunc, Roskilde, Dänemark, Artikel Nr. 262162) wurden mit Anti-HSV 1 und 2 Immunglobulin G (IgG) von der Ziege beschichtet.
Dazu wurde das IgG auf 20 mg/l in 100 mmol/l Natriumbicarbonat pH 9,6 verdünnt. In jede Vertiefung (Well) der Mikrotestplatten wurden 100 µl der Verdünnung gegeben. Die derart gefüllten Testplatten wurden 18 Stunden bei 20°C belassen, dann wurden die Lösungen in den Wells abgesaugt und die Wells 3-4 mal mit 200 µl einer Lösung von 1 g/l ^{R}Tween 20 in phosphatgepufferter physiologischer Kochsalzlösung, pH 7,4 durch Füllen und Absaugen gewaschen und die Testplatten anschließend über Kieselgel bei 20°C getrocknet.

### 1.2. Anti-HSV 1 und 2 IgG-Peroxidase-Konjugat

Monoklonales Maus-IgG, das sowohl gegen HSV 1 als auch gegen HSV 2 gerichtet war, wurde mit N-gamma-Maleimidobutylyloxisuccinimid (GMBS) umgesetzt wie von Tanamori et al., 1983 in J. Immunol. Meth. 62, 123-131 beschrieben. 2-Iminothiolanhydrochlorid (Fa. Sigma, Kat. Nr. I 6256) wurde mit Meerrettich-Peroxidase (POD), bezogen von der Fa. Boehringer Mannheim, Kat. Nr. 413470, umgesetzt wie von King et al. 1978 in Biochem. 17, 1499-1506 beschrieben. Aus dem GMBS-IgG Konjugat und dem Iminothiolan-POD Konjugat wurde ein IgG-POD Konjugat hergestellt wie von Tanamori beschrieben.

Die erhaltene Lösung des IgG-POD Konjugats hatte einen Proteingehalt von 1,2 mg/ml. Das Verhältnis von POD zu IgG wurde mit 2.5 bestimmt. Die Lösung wurde anschliessend auf 6 µg/ml IgG-POD mit einer Lösung von 50 ml/l foetalem Kälberserum, 5 g/l ^{R}Tween 20 in PBS verdünnt und erhielt die Bezeichnung Anti-HSV-POD.

### 1.3. TMB-Substratzubereitung

Zum Nachweis von Anti-HSV-POD wurde ein Substratsystem oder eine Substratzubereitung verwandt, enthaltend Wasserstoffperoxid und Tetramethylbenzidin (TMB), welche aus zwei Stammlösungen hergestellt wurde.

Stammlösung 1: TMB-Dihydrochlorid wurde unter Rühren in einer Konzentration von 5 g/l, d. h. von 16 mmol/l in bidestilliertem Wasser gelöst und mit 5 normaler Salzsäure auf pH 1.5 eingestellt. Zu dieser Lösung wurde Penicillin G unter Rühren in einer Endkonzentration von 200 mg/l, d.h. von 0.56 mmol/l zugesetzt.

Stammlösung 2: zu 900 ml bidestilliertem Wasser wurde 1.4 ml Eisessig, 1.5 ml 1 normale NaOH und 250 mg, d.h. 3 mmol H₂O₂ als Harnstoff-Wasserstoffperoxid-Addukt zugesetzt. Nach vollständigem Lösen wurde mit bidestilliertem Wasser auf 1 l aufgefüllt.

TMB-Substratzubereitung: Ein Volumenteil der Stammlösung 1 und 10 Volumenteile der Stammlösung 2 wurden miteinander vermischt.

### 1.4. Durchführung der Bestimmung

Jeweils 150 µl der zuvor beschriebenen aus Bläschenflüssigkeit hergestellten HSV-haltigen Proben und 150 µl der für Aufbereitung benutzten jeweiligen Lösungen als Leerwert wurden in Wells der Anti-HSV-Testplatten (Beispiel 1.1.) eingefüllt und 2 h bei 37°C in einem Brutschrank belassen. Der In- halt der Wells wurde durch Absaugen entfernt und die Wells mit einer als Waschpuffer bezeichneten Lösung von 1 g/l ^{R}Tween 20 in PBS viermal gewaschen.

Anschließend wurde in jede Well 100 µl Anti-HSV-POD gegeben und 1 h bei 37°C inkubiert wie zuvor beschrieben. Der Inhalt der Wells wurde durch Absaugen entfernt und die Wells viermal mit Waschpuffer gewaschen. Es wurden in jede Well 100 µl TMB-Substratzubereitung gegeben, 30 min bei 20-22°C inkubiert und die Inkubation durch Zugabe von 100 µl 1 normale Schwefelsäure beendet. E₄₅₀ der gefärbten Lösung wurde gegen einen Leerwert von PBS gemessen.

Die Ergebnisse sind in der Tabelle dargestellt. Aus ihr wird ersichtlich, daß die Bläschenflüssigkeit nach Behandlung mit den Lösungen, die ein Aminoxid enthalten, deutlich höhere Extinktionen zeigt , als Bläschenflüssigkeiten, die mit Lösungen von Pluronic F 68, von Tetronic 707 und von Tetronic 1107, also Lösungen des Standes der Technik behandelt worden waren.

**TABELLE**

| Behandlung von HSV-haltiger Bläschenflüssigkeit mit Lösungen der genannten Zusätze in PBS | | deltaExtinktion bei 450 nm (E_{Probe} -E_{Leerwert}) | | | |
|---|---|---|---|---|---|
| Auswertungen an den Tagen | | 0 | 7 | 14 | 21 |

| Zusätze | | | | | |
|---|---|---|---|---|---|
| Aminoxid WS35 | (a) | 619 | n.t. | 644 | 653 |
| | (b) | 578 | 590 | 658 | 637 |
| Cocos-dimethylaminoxid | (a) | 661 | 582 | 577 | 611 |
| | (b) | 721 | 577 | 547 | 554 |
| Tetradecyldimethylaminoxid | (a) | 676 | 551 | 530 | 549 |
| | (b) | 580 | 506 | 461 | 524 |
| Cocos-bis (2-hydroxyethyl)aminoxid | (a) | 688 | 629 | 581 | 565 |
| | (b) | 646 | 560 | 605 | 419 |
| Pluronic F 68 | (a) | 386 | | | |
| | (b) | 351 | | | |
| Tetronic 707 | (a) | 374 | | | |
| | (b) | 320 | | | |
| Tetronic 1107 | (a) | 385 | | | |
| | (b) | 336 | | | |

### Beispiel 2

Es wurden Lösungen von
a) 10 g/l N-Acetyl-L-Cystein
b) 1 g/l Pancreatin und c) 40 ml/l Aminoxid WS35 in PBS enthaltend 40 ml/l foetales Kälberserum hergestellt.

Die Lösungen wurden zu gleichen Teilen mit abgehustetem Sputum vermischt, bei 18 bis 25°C gehalten und in Abständen von 10 min auf Pipettierbarkeit bei Verwendung einer 200 µl Eppendorfpipette geprüft.

Es stellte sich heraus, daß die Mischung mit Lösung c) nach 30 min pipettierbar und somit als Probe verwendbar wurde, während die Mischungen mit den Lösungen a) und b) nach dem genannten Zeitpunkt noch nicht pipettiert werden konnten.

## Patentansprüche (Patentansprüche für folgende(n) Vertragsstaat(en): AT, BE, CH, DE, FR, GB, IT, LI, LU, NL, SE)

1. Mittel zum Nachweis und zur Bestimmung eines Analyten in einem biologischen Material, enthaltend ein Aminoxid der Formel I 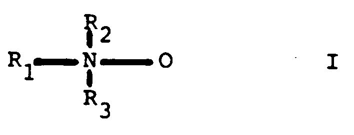 wobei
a) R₁ eine 1-Alkanoyl- oder 1-Alkenoyl-amino-propyl-Gruppe ist, wobei die Alkanoyl- oder Alkenoyl-Reste 8-18 C-Atome enthalten oder deren Mischungen und
R₂ und R₃ Methyl-, Ethyl- oder Propyl-Gruppen oder deren Mischungen sind, oder
b) R₁ eine Alkanyl- oder Alkenyl-Gruppe mit 8 bis 18 C-Atomen oder deren Mischungen und
R₂ und R₃ Methyl-, Ethyl- oder Propyl-Gruppen oder deren Mischungen sind, oder
c) R₁ eine Alkanyl- oder Alkenyl-Gruppe mit 8 bis 18 C-Atomen oder deren Mischungen,
R₂ eine Methyl-, Ethyl- oder Propyl-Gruppe ist, und
R₃ eine Hydroxiethyl- oder Hydroxipropyl-Gruppe ist, oder deren Mischungen, oder
d) R₁ eine Alkanyl- oder Alkenyl-Gruppe mit 8 bis 18 C-Atomen oder deren Mischungen und
R₂ und R₃ Hydroxiethyl- oder Hydroxipropyl-Gruppen sind, oder
e) R₁ eine Alkanyl- oder Alkenyl-Gruppe mit 8 bis 18 C-Atomen oder deren Mischungen und Oxiethyl- oder Oxipropyl-Gruppe oder deren Polykondensaten,
R₂ und R₃ Methyl-, Ethyl-, Propyl- oder Hydroxiethyl- oder Hydroxipropyl-Gruppen oder Mischungen davon sind, oder
f) R₁ eine Alkanoyl- oder Alkenoyl-Gruppen mit 8-18 C-Atomen oder deren Mischungen und Oxiethyl- oder Oxipropyl-Gruppe oder deren Polykondensaten,
R₂ und R₃ Methyl-, Ethyl-, Propyl- oder Hydroxiethyl- oder Hydroxipropyl-Gruppen oder Mischungen davon sind,
wobei R₁, R₂ und R₃ Amidgruppen oder Phenolreste enthalten können, und immunchemische Reaktanten, von denen mindestens einer mit dem Analyten reagieren kann.

2. Mittel nach Anspruch 1, dadurch gekennzeichnet, daß das Aminoxid 1-Alkoxylamino-3-dimethylamino-propan-3-N-oxid ist, wobei sich der Alkoxylrest aus Mischungen von Fettsäuren mit Kettenlängen von C₈ bis C₁₈ herleitet.

3. Mittel nach Anspruch 1, dadurch gekennzeichnet, daß R₁ ein Cocosfett erhaltenes Alkyl ist, R₂ und R₃ Methyl sind.

4. Mittel nach Anspruch 1, dadurch gekennzeichnet, daß das Aminoxid Tetradecyldimethylaminoxid ist.

5. Mittel nach Anspruch 1, dadurch gekennzeichnet, daß R₁ und R₂ aus Cocosfett erhaltene Alkyle sind und R₃ 2-Hydroxyethyl ist.

6. Mittel nach Anspruch 1, dadurch gekennzeichnet, daß sich das Aminoxid in einem Gefäß befindet.

7. Mittel nach Anspruch 1, dadurch gekennzeichnet, daß das Aminoxid in einer Lösung gelöst oder suspendiert ist.

8. Mittel nach Anspruch 1, dadurch gekennzeichnet, daß sich das Aminoxid in einem trockenchemischen Testsystem befindet.

9. Verfahren zur immunchemischen Bestimmung eines in einem biologischen Material enthaltenen Analyten dadurch gekennzeichnet, daß man das biologischen Material mit einem Aminoxid nach Formel I des Anspruchs 1, gegebenenfalls in einer wässrigen Lösung, zusammenbringt und mit dem derart erhaltenen Gemisch eine immunchemische Bestimmung durchführt.

10. Verfahren nach Anspruch 9, dadurch gekennzeichnet, daß man einen in fester Phase vorliegenden immunchemischen Reaktanten mit dem biologischen Material und dem Aminoxid in flüssiger Phase, gegebenenfalls mit weiteren immunchemischen Reaktanten, zusammenbringt, anschließend die feste Phase von der flüssigen Phase trennt und den Analyten entweder an der festen Phase oder in der flüssigen Phase bestimmt.

11. Verwendung eines Aminoxids nach Formel I des Anspruchs 1 in immunchemischen Tests.

## Patentansprüche (Patentansprüche für folgende(n) Vertragsstaat(en): ES)

1. Verfahren zur immunchemischen Bestimmung eines in einem biologischen Material enthaltenen Analyten dadurch gekennzeichnet, daß man den Analyten mit einem Aminoxid der Formel I 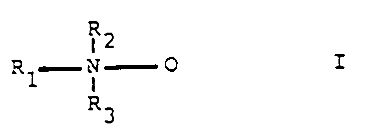 wobei
a) R₁ 1-Alkanoyl- oder 1-Alkenoyl-amino-propyl-Gruppen sind, wobei die Alkanoyl- oder Alkenoyl-Reste 8-18 C-Atome enthalten oder deren Mischungen und
R₂ und R₃ Methyl-, Ethyl- oder Propyl-Gruppen oder deren Mischungen sind, oder
b) R₁ Alkanyl- oder Alkenyl-Gruppen mit 8 bis 18 C-Atomen oder deren Mischungen sind, oder
c) R₁ Alkanyl- oder Alkenyl-Gruppen mit 8 bis 18 C-Atomen oder deren Mischungen,
R₂ Methyl-, Ethyl- oder Propyl- und
R₃ Hydroxiethyl- oder Hydroxipropyl- oder deren Mischungen sind, oder
d) R₁ Alkanyl- oder Alkenyl-Gruppen mit 8 bis 18 C-Atomen oder deren Mischungen und
R₂ und R₃ Hydroxiethyl- oder Hydroxipropyl- sind, oder
e) R₁ Alkanyl- oder Alkenyl-Gruppen mit 8 bis 18 C-Atomen oder deren Mischungen und Oxiethyl-oder Oxipropyl- oder deren Polykondensaten,
R₂ und R₃ Methyl-, Ethyl-, Propyl- oder Hydroxiethyl-oder Hydroxipropyl- oder Mischungen davon sind, oder
f) R₁ Alkanoyl- oder Alkenoyl-Gruppen mit 8-18 C-Atomen oder deren Mischungen und Oxiethyl- oder Oxipropyl-oder deren Polykondensaten,
R₂ und R₃ Methyl-, Ethyl-, Propyl- oder Hydroxiethyl oder Hydroxipropyl- oder Mischungen davon sind,
wobei R₁, R₂ und R₃ Amidgruppen oder Phenolreste enthalten können, ist, gegebenenfalls in einer wässrigen Lösung zusammenbringt und mit dem derart erhaltenen Gemisch eine immunchemische Bestimmung durchführt.

2. Verfahren nach Anspruch 1, dadurch gekennzeichnet, daß man einen in fester Phase vorliegenden immunchemischen Reaktanten mit dem biologischen Material und dem Aminoxid in flüssiger Phase, gegebenenfalls mit weiteren immunchemischen Reaktanten, zusammenbringt, anschließend die feste Phase von der flüssigen Phase trennt und den Analyten entweder an der festen Phase oder in der flüssigen Phase bestimmt.

## Claims (Claims for the following Contracting State(s): AT, BE, CH, DE, FR, GB, IT, LI, LU, NL, SE)

1. An agent, for the detection and for the determination of an analyte in a biological material, containing an amine oxide of the formula I 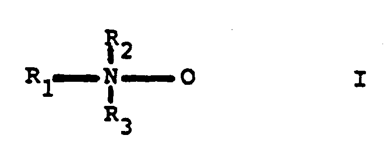 where
a) R₁ is a 1-alkanoyl group or 1-alkenoyl-aminopropyl group where the alkanoyl radicals or alkenoyl radicals contain 8-18 carbon atoms or their mixtures and
R₂ and R₃ are methyl groups, ethyl groups or propyl groups or their mixtures, or
b) R₁ is an alkanyl group or alkenyl group having 8 to 18 carbon atoms or their mixtures and
R₂ and R₃ are methyl groups, ethyl groups or propyl groups or their mixtures, or
c) R₁ is an alkanyl group or alkenyl group having 8 to 18 carbon atoms or their mixtures,
R₂ is a methyl group, ethyl group or propyl group and
R₃ is a hydroxyethyl group or hydroxypropyl group or their mixtures, or
d) R₁ is an alkanyl group or alkenyl group having 8 to 18 carbon atoms or their mixtures and
R₂ and R₃ are hydroxyethyl groups or hydroxypropyl groups, or
e) R₁ is an alkanyl group or alkenyl group having 8 to 18 carbon atoms or their mixtures and oxyethyl group or oxypropyl group or their polycondensates,
R₂ and R₃ are methyl groups, ethyl groups, propyl groups or hydroxyethyl groups or hydroxypropyl groups or mixtures thereof, or
f) R₁ is an alkanoyl group or alkenoyl group having 8-18 carbon atoms or their mixtures and oxyethyl group or oxypropyl group or their polycondensates,
R₂ and R₃ are methyl groups, ethyl groups, propyl groups or hydroxyethyl groups or hydroxypropyl groups or mixtures thereof, where R₁, R₂ and R₃ may contain amido groups or phenol radicals, and immunochemical reactants, of which at least one can react with the analyte.

2. An agent as claimed in claim 1, wherein the amine oxide is 1-alkoxylamino-3-dimethylamino-propane-3-N-oxide where the alkoxyl radical is derived from mixtures of fatty acids having chain lengths of from C₈ to C₁₈.

3. An agent as claimed in claim 1, wherein R₁ is an alkyl obtained from coconut fat, and R₂ and R₃ are methyl.

4. An agent as claimed in claim 1, wherein the amine oxide is tetradecyldimethylamine oxide.

5. An agent as claimed in claim 1, wherein R₁ and R₂ are alkyls obtained from coconut fat and R₃ is 2-hydroxyethyl.

6. An agent as claimed in claim 1, wherein the amine oxide is present in a vessel.

7. An agent as claimed in claim 1, wherein the amine oxide is dissolved or suspended in a solution.

8. An agent as claimed in claim 1, wherein the amine oxide is present in a dry-chemical assay system.

9. A process for the immunochemical determination of an analyte contained in a biological material, which comprises combining the biological material with an amine oxide of formula I of claim 1, if appropriate in an aqueous solution, and carrying out an immunochemical determination on the resultant mixture.

10. The process as claimed in claim 9, wherein an immunochemical reactant which is present in solid phase is combined with the biological material and the amine oxide in liquid phase, if appropriate with other immunochemical reactants, the solid phase is subsequently separated from the liquid phase, and the analyte is determined either on the solid phase or in the liquid phase.

11. Use of an amine oxide of formula I of claim 1 in immunochemical assays.

## Claims (Claims for the following Contracting State(s): ES)

1. A process for the immunochemical determination of an analyte contained in a biological material, which comprises combining the analyte with an amine oxide of the formula I 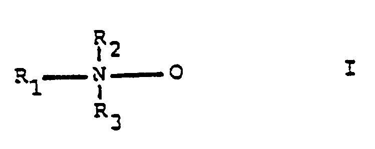 where
a) R₁ is 1-alkanoyl groups or 1-alkenoyl-aminopropyl groups where the alkanoyl radicals or alkenoyl radicals contain 8-18 carbon atoms or their mixtures and
R₂ and R₃ are methyl groups, ethyl groups or propyl groups or their mixtures, or
b) R₁ is alkanyl groups or alkenyl groups having 8 to 18 carbon atoms or their mixtures, or
c) R₁ is alkanyl groups or alkenyl groups having 8 to 18 carbon atoms or their mixtures,
R₂ is methyl, ethyl or propyl and
R₃ is hydroxyethyl or hydroxypropyl or their mixtures, or
d) R₁ is alkanyl groups or alkenyl groups having 8 to 18 carbon atoms or their mixtures and
R₂ and R₃ are hydroxyethyl or hydroxypropyl, or
e) R₁ is alkanyl groups or alkenyl groups having 8 to 18 carbon atoms or their mixtures and oxyethyl or oxypropyl or their polycondensates,
R₂ and R₃ are methyl, ethyl, propyl or hydroxyethyl or hydroxypropyl or mixtures thereof, or
f) R₁ is alkanoyl groups or alkenoyl groups having 8-18 carbon atoms or their mixtures and oxyethyl or oxypropyl or their polycondensates,
R₂ and R₃ are methyl, ethyl, propyl or hydroxyethyl or hydroxypropyl or mixtures thereof, where R₁, R₂ and R₃ may contain amido groups or phenol radicals, if appropriate in an aqueous solution, and carrying out an immunochemical determination on the resultant mixture.

2. The process as claimed in claim 1, wherein an immunochemical reactant which is present in solid phase is combined with the biological material and the amine oxide in liquid phase, if appropriate with other immunochemical reactants, the solid phase is subsequently separated from the liquid phase, and the analyte is determined either on the solid phase or in the liquid phase.

## Revendications (Revendications pour l'(les) Etat(s) contractant(s) suivant(s): AT, BE, CH, DE, FR, GB, IT, LI, LU, NL, SE)

1. Nécessaire pour la détection et pour la détermination d'un corps à analyser dans un produit biologique, contenant un oxyde d'amine de formule I 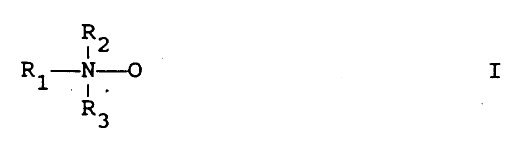 dans laquelle
a) R₁ est un groupe 1-alcanoyl- ou 1-alcénoylaminopropyle, les radicaux alcanoyle ou alcénoyle contenant de 8 à 18 atomes de carbone, ou des mélanges de ceux-ci, et
R₂ et R₃ sont des groupes méthyle, éthyle ou propyle, ou des mélanges de ceux-ci, ou
b) R₁ est un groupe alkyle ou alcényle ayant de 8 à 18 atomes de carbone, ou des mélanges de ceux-ci, et
R₂ et R₃ sont des groupes méthyle, éthyle ou propyle, ou des mélanges de ceux-ci, ou
c) R₁ est un groupe alkyle ou alcényle ayant de 8 à 18 atomes de carbone, ou des mélanges de ceux-ci,
R₂ est un groupe méthyle, éthyle ou propyle, et
R₃ est un groupe hydroxyéthyle ou hydroxypropyle ou des mélanges de ceux-ci, ou
d) R₁ est un groupe alkyle ou alcényle ayant de 8 à 18 atomes de carbone, ou des mélanges de ceux-ci, et
R₂ et R₃ sont des groupes hydroxyéthyle ou hydroxypropyle, ou
e) R₁ est un groupe alkyle ou alcényle ayant de 8 à 18 atomes de carbone, ou des mélanges de ceux-ci, et un groupe oxyéthyle ou oxypropyle ou leurs produits de polycondensation,
R₂ et R₃ sont des groupes méthyle, éthyle, propyle, hydroxyéthyle ou hydroxypropyle, ou des mélanges de ceux-ci, ou
f) R₁ est un groupe alcanoyle ou alcénoyle ayant de 8 à 18 atomes de carbone, ou des mélanges de ceux-ci, et un groupe oxyéthyle ou oxypropyle ou leurs produits de polycondensation,
R₂ et R₃ sont des groupes méthyle, éthyle, propyle ou hydroxyéthyle ou hydroxypropyle, ou des mélanges de ceux-ci,
R₁, R₂ et R₃ pouvant contenir des groupes amido ou des radicaux phénol,
et des réactifs immunochimiques, dont au moins un peut réagir avec l'analyte

2. Nécessaire selon la revendication 1, caractérisé en ce que l'oxyde d'amine est un N-oxyde de 1-alcoxyamino-3-diméthylamino-propane-3, le radical alcoxy dérivant de mélanges d'acides gras à longueurs de chaînes allant de C₈ à C₁₈.

3. Nécessaire selon la revendication 1, caractérisé en ce que R¹ est un radical alkyle obtenu à partir de l'huile de coprah, et R² et R³ sont des groupes méthyle.

4. Nécessaire selon la revendication 1, caractérisé en ce que l'oxyde d'amine est l'oxyde de tétradécyldiméthylamine.

5. Nécessaire selon la revendication 1, caractérisé en ce que R¹ et R² sont des radicaux alkyle obtenus à partir de l'huile de coprah, et R³ est le groupe 2-hydroxyéthyle.

6. Nécessaire selon la revendication 1, caractérisé en ce que l'oxyde d'amine se trouve dans un récipient.

7. Nécessaire selon la revendication 1, caractérisé en ce que l'oxyde d'amine est dissous ou mis en suspension dans une solution.

8. Nécessaire selon la revendication 1, caractérisé en ce que l'oxyde d'amine se trouve dans un système d'essai chimique sec.

9. Procédé pour la détermination immunochimique d'un produit biologique contenant des corps à analyser, caractérisé en ce que l'on réunit le produit biologique avec un oxyde d'amine correspondant à la formule I de la revendication 1, éventuellement dans une solution aqueuse, et on effectue une détermination immunochimique avec le mélange ainsi obtenu.

10. Procédé selon la revendication 9, caractérisé en ce que l'on réunit en phase liquide des réactifs immunochimiques se trouvant en phase solide, avec le produit biologique et l'oxyde d'amine, et éventuellement avec d'autres réactifs immunochimiques, ensuite on sépare la phase solide de la phase liquide et on détermine le corps à analyser soit sur la phase solide, soit dans la phase liquide.

11. Utilisation d'un oxyde d'amine correspondant à la formule I de la revendication 1, dans un essai immunochimique.

## Revendications (Revendications pour l'(les) Etat(s) contractant(s) suivant(s): ES)

1. Procédé pour la détermination immunochimique d'un corps à analyser contenu dans un produit biologique, caractérisé en ce que l'on met en contact le corps à analyser avec un oxyde d'amine de formule I 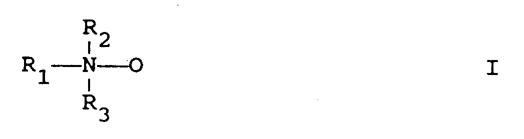 dans laquelle
a) R₁ est un groupe 1-alcanoyl- ou 1-alcénoylaminopropyle, les radicaux alcanoyle ou alcénoyle contenant de 8 à 18 atomes de carbone, ou des mélanges de ceux-ci, et
R₂ et R₃ sont des groupes méthyle, éthyle ou propyle, ou des mélanges de ceux-ci, ou
b) R₁ est un groupe alkyle ou alcényle ayant de 8 à 18 atomes de carbone, ou des mélanges de ceux-ci, et
R₂ et R₃ sont des groupes méthyle, éthyle ou propyle, ou des mélanges de ceux-ci, ou
c) R₁ est un groupe alkyle ou alcényle ayant de 8 à 18 atomes de carbone, ou des mélanges de ceux-ci,
R₂ est un groupe méthyle, éthyle ou propyle, et
R₃ est un groupe hydroxyéthyle ou hydroxypropyle ou des mélanges de ceux-ci, ou
d) R₁ est un groupe alkyle ou alcényle ayant de 8 à 18 atomes de carbone, ou des mélanges de ceux-ci, et
R₂ et R₃ sont des groupes hydroxyéthyle ou hydroxypropyle, ou
e) R₁ est un groupe alkyle ou alcényle ayant de 8 à 18 atomes de carbone, ou des mélanges de ceux-ci, et un groupe oxyéthyle ou oxypropyle ou leurs produits de polycondensation,
R₂ et R₃ sont des groupes méthyle, éthyle, propyle, hydroxyéthyle ou hydroxypropyle, ou des mélanges de ceux-ci, ou
f) R₁ est un groupe alcanoyle ou alcénoyle ayant de 8 à 18 atomes de carbone, ou des mélanges de ceux-ci, et un groupe oxyéthyle ou oxypropyle ou leurs produits de polycondensation,
R₂ et R₃ sont des groupes méthyle, éthyle, propyle ou hydroxyéthyle ou hydroxypropyle, ou des mélanges de ceux-ci,
R₁, R₂ et R₃ pouvant contenir des groupes amido ou des radicaux phénol,
éventuellement dans une solution aqueuse, et on effectue une détermination immunochimique avec le mélange ainsi obtenu.

2. Procédé selon la revendication 1, caractérisé en ce que l'on met en contact en phase liquide un réactif immunochimique se trouvant en phase solide, avec le produit biologique et l'oxyde d'amine, et éventuellement avec d'autres réactifs immunochimiques, ensuite on sépare la phase solide de la phase liquide et on détermine le corps a analyser soit sur la phase solide, soit dans la phase liquide.
